# EUROPEAN PATENT APPLICATION

(11) **EP 3 560 930 A1**
(43) Date of publication of application: **30.10.2019**
(21) Application number: 17886868.3
(22) Date of filing: 21.12.2017
(51) Int. Cl.: C07D 495/04, A61K 31/519, A61K 31/496

(54) **NOVEL METHOD FOR PREPARING THIENOPYRIMIDINE COMPOUND AND INTERMEDIATE**

(30) Priority: 26.12.2016 KR 20160179320
(71) Applicant: Hanmi Pharm. Co., Ltd., Hwaseong-si, Gyeonggi-do 18536 (KR)
(72) Inventor: KIM, Hee Cheol, Hwaseong-si Gyeonggi-do 18469 (KR); HA, Tae Hee, Hwaseong-si Gyeonggi-do 18469 (KR); SUH, Kwee Hyun, Hwaseong-si Gyeonggi-do 18469 (KR)
(74) Representative: Brevalex
(86) International application number: PCT/KR2017/015297
(87) International publication number: WO 2018/124644

(57) **Abstract**

Provided are a novel method of preparing a thienopyrimidine compound having activity of selectively inhibiting tyrosine kinase, particularly, mutant epidermal growth factor receptor tyrosine kinase, and a novel intermediate used for the novel method. According to the method of the present disclosure, a compound of Formula 1 useful as a therapeutic agent for non-small cell lung cancer induced by mutant epidermal growth factor receptor tyrosine kinase can be industrially mass-produced more easily and efficiently than the prior art.

## Description

### TECHNICAL FIELD

The present disclosure relates to a novel method of preparing a thienopyrimidine compound having activity of selectively inhibiting tyrosine kinase, particularly, mutant epidermal growth factor receptor tyrosine kinase, and a novel intermediate used for the novel method.

### BACKGROUND ART

US 8,957,065 and WO 2011/162515 each disclose a thienopyrimidine compound of Formula 1 having activity of selectively inhibiting mutant epidermal growth factor receptor tyrosine kinase.

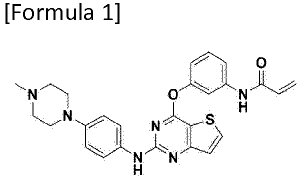

In addition, these documents each disclose a process of preparing the compound of Formula 1. In particular, as shown in Reaction Scheme 1, 2,4-dichlorothieno[3,2-d]pyrimidine of Formula A is reacted with 3-nitrophenol to produce a compound of Formula B, the compound of Formula B is reacted with 4-(4-methylpiperazine-1-yl)aniline to produce a compound of Formula C, a nitro group of the compound of Formula C is reduced to produce a compound of Formula D, and the compound of Formula D is reacted with acryloyl chloride to produce a compound of Formula 1.

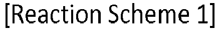

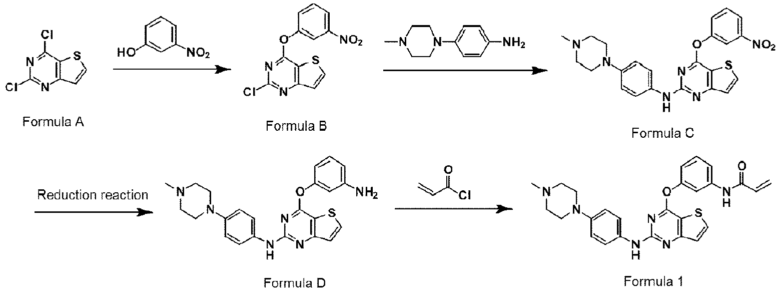

However, a synthesis method through Reaction Scheme 1 has the following problems in the formulation. To remove impurities generated in the step of obtaining the compound of Formula C, a column chromatography purification method is used, but this method is not suitable for industrial production and has a low yield. In addition, to reduce the nitro group of the compound of Formula C, an excess of iron or an additional reaction with hydrogen in the presence of a metal catalyst is required. In addition, it is difficult to purify both the compound of Formula D and the compound of Formula 1 by an unsuitable column chromatography method which is difficult to apply to industrial production.

In this regard, the present inventors have completed the present disclosure by developing a novel method of preparing a thienopyrimidine compound, which is easy to manufacture, efficient, and industrially applicable.

### DESCRIPTION OF EMBODIMENTS

### TECHNICAL PROBLEM

Provided is a method of preparing a thienopyrimidine compound conveniently and efficiently.

Provided is an intermediate used for the method of preparing the thienopyrimidine compound.

### SOLUTION TO PROBLEM

According to an aspect of the present disclosure, there is provided a method of preparing a compound of Formula 1, the method including: 1) obtaining a compound of Formula 3 through a reaction between a compound of Formula 4 and a compound of Formula 5 in the presence of a base in an organic solvent; 2) obtaining a compound of Formula 2 through a reaction between the compound of Formula 3 and acid in an organic solvent; and 3) performing a reaction between the compound of Formula 2 and acryloyl chloride in the presence of a base in an organic solvent: 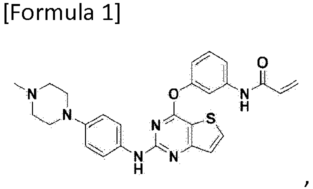 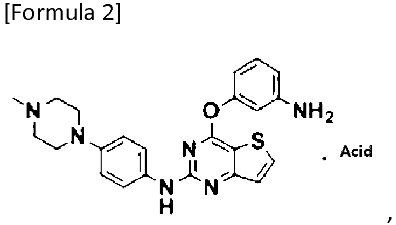 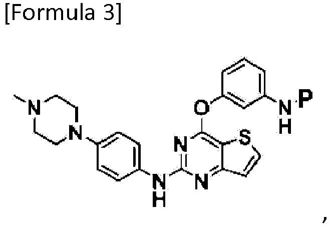 (wherein, P in Formula 3 represents an amino group-protecting group), 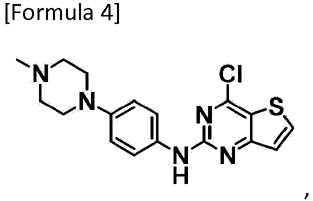 and 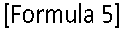 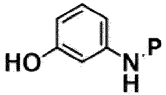 (wherein, P in Formula 5 represents an amino group-protecting group).

According to another aspect of the present disclosure, there is provided a compound of Formula 2:

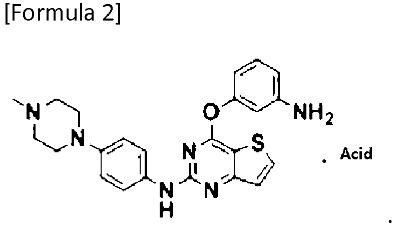

According to another aspect of the present disclosure, there is provided a compound of Formula 3: 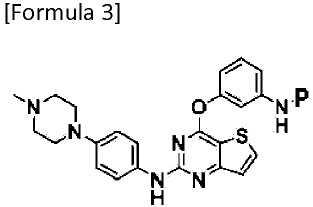 (wherein, P in Formula 3 represents an amino group-protecting group).

### ADVANTAGEOUS EFFECTS OF DISCLOSURE

According to the method of the present disclosure, a compound of Formula 1 that is useful as a therapeutic agent for non-small cell lung cancer induced by mutant epidermal growth factor receptor tyrosine kinase can be industrially mass-produced more easily and efficiently than the prior art.

### MODE OF DISCLOSURE

All technical terms used in the present disclosure are, unless otherwise defined, used in the sense that these terms are generally understood by one of ordinary skill in the relevant field of the present disclosure. In addition, preferred methods or samples are described in the present specification, but similar or equivalent methods of samples are also included in the scope of the present disclosure. In addition, although not explicitly stated, the numerical values set forth herein are considered to include the meaning of "about". The contents of all publications referred to the present specification are incorporated herein by reference in their entirety.

Hereinafter, the present disclosure will be described in detail.

An aspect of the present disclosure provides a method of preparing a compound of Formula 1, the method including: 1) obtaining a compound of Formula 3 through a reaction between a compound of Formula 4 and a compound of Formula 5 in the presence of a base in an organic solvent; 2) obtaining a compound of Formula 2 through a reaction between the compound of Formula 3 and acid in an organic solvent; and 3) performing a reaction between the compound of Formula 2 and acryloyl chloride in the presence of a base in an organic solvent: 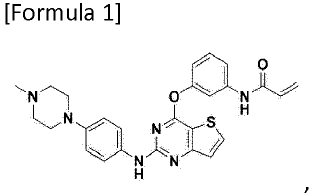 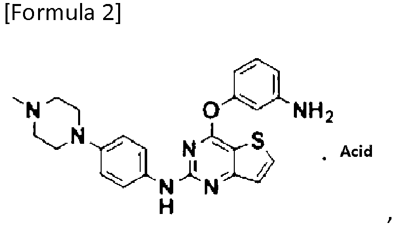 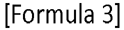 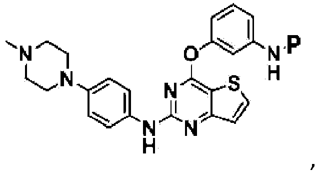 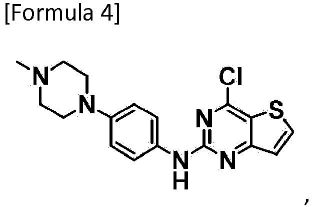 and 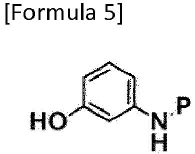 (wherein, P in Formulae 3 and 5 indicates an amino group-protecting group).

In one embodiment, P in Formulae 3 and Formula 5 may be a tert-butyloxycarbonyl group (Boc) or a benzyloxycarbonyl group (Cbz).

Another aspect of the present disclosure provides a compound of Formula 2:

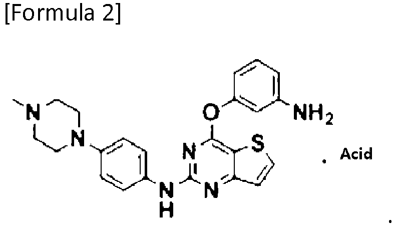

The compound of Formula 2 may be an acid salt of 4-(3-aminophenoxy)-N-(4-(4-methylpiperazine-1-yl)phenyl)thieno[3,2-d]pyrimidine-2-amine. The acid in Formula 2 may be inorganic acid or organic acid. The inorganic acid may be selected from the group consisting of hydrochloric acid, nitric acid, sulfuric acid, and any combination thereof. In addition, the organic acid may be selected from the group consisting of toluenesulfonic acid, benzene sulfonic acid, methanesulfonic acid, acetic acid, trifluoroacetic acid, and any combination thereof. In one embodiment, the acid in Formula 2 may be hydrochloric acid or trifluoroacetic acid.

In addition, another aspect of the present disclosure provides a compound of Formula 3:

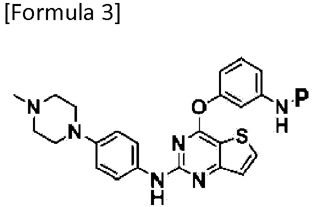

In Formula 3, P indicates an amino group-protecting group. In one embodiment, P may be a Boc group or a Cbz group.

Hereinafter, steps 1) to 3) of the preparation method according to the present disclosure will be described in more detail

The preparation method according to the present disclosure includes **step 1)** of obtaining a compound of Formula 3 through a reaction between a compound of Formula 4 and a compound of Formula 5 in the presence of a base in an organic solvent.

In one embodiment, the compound of Formula 3 may be t-butyl(3-((2-((4-(4-methylpiperazine-1-yl)phenyl)amino)thieno[3,2-d]pyrimidine-4-yl)oxy)phenyl)carbamate or benzyl(3-((2-((4-(4-methylpiperazine-1-yl)phenyl)amino)thieno[3,2-d]pyrimidine-4-yl)oxy)phenyl)carbamate.

In one embodiment regarding step 1), 4-chloro-N-(4-(4-methylpiperazine-1-yl)phenyl)thieno[3,2-d]pyrimidine-2-amine, which is the compound of Formula 4, is reacted with t-butyl (3-hydroxyphenyl)carbamate, which is an example of the compound of Formula 5, in the presence of a base in an organic solvent. Then, water or a mixed solvent of an organic solvent and water is added thereto for crystallization, and the resulting product is filtered to obtain t-butyl(3-((2-((4-(4-methylpiperazine-1-yl)phenyl)amino)thieno[3,2-d]pyrimidine-4-yl)oxy)phenyl)carbamate, which is an example of the compound of Formula 3.

In one or more embodiments regarding step 1), 4-chloro-N-(4-(4-methylpiperazine-1-yl)phenyl)thieno[3,2-d]pyrimidine-2-amine, which is the compound of Formula 4, is reacted with benzyl (3-hydroxyphenyl)carbamate, which is an example of the compound of Formula 5, in the presence of a base in an organic solvent. Then, water or a mixed solvent of an organic solvent and water is added thereto for crystallization, and the resulting product is filtered to obtain benzyl(3-((2-((4-(4-methylpiperazine-1-yl)phenyl)amino)thieno[3,2-d]pyrimidine-4-yl)oxy)phenyl)carbamate, which is an example of the compound of Formula 3.

The organic solvent used in the reaction with the compound of Formula 5 may be selected from the group consisting of acetonitrile, acetone, methyl ethyl ketone, methyl isobutyl ketone, methyl acetate, ethyl acetate, isopropyl acetate, N'N-dimethylformamide, N'N-dimethylacetamide, dimethylsulfoxide, and any combination thereof. In one embodiment, the organic solvent may be dimethylsulfoxide.

An amount of the organic solvent may be in a range of about 5 ml to about 20 ml, for example, about 10 ml to about 15 ml, per 1 g of the compound of Formula 4.

In addition, the base used in the reaction above may be potassium carbonate, calcium carbonate, sodium carbonate, potassium hydroxide, sodium hydroxide, sodium bicarbonate, or any mixture thereof. In one embodiment, the base may be potassium carbonate or sodium carbonate.

An amount of the base may be in a range of about 1.0 mole to about 3.0 moles, for example, about 1.0 mole to about 2.0 moles, per equivalent weight of 1 mole of the compound of Formula 4.0 moles, per equivalent weight of 1 mole of the compound of Formula 4.

The reaction above may be performed at a temperature in a range of about 10 °C to about 100 °C, for example, about 40 °C to about 70 °C, for about 3 hours to about 12 hours, for example, about 4 hours to about 8 hours.

The compound of Formula 3 resulting from step 1) may be obtained in the form of a crystal by adding water or a mixed solvent of an organic solvent and water to a reactant in which the compound of Formula 3 is present and precipitating the resulting product in a solid state.

The organic solvent used for the crystallization may be an organic solvent mixable with water, and for example, may be selected from the group consisting of acetone, methanol, ethanol, isopropanol, acetonitrile, and any combination thereof. In one embodiment, the organic solvent may be acetone or isopropanol.

A mixing ratio of the organic solvent and water may vary depending on the type of the organic solvent. In one embodiment, when the organic solvent is acetone or isopropanol, the mixing ratio of the organic solvent and water may be in a range of about 1:10 to about 10:1, for example, about 3:1 to about 1:3, by volume. In one embodiment, the mixing ratio may be in a range of about 1:1 to about 1:2 by volume.

An amount of the mixed solvent of the organic solvent and water used for the crystallization may be in a range of about 10 ml to about 50 ml, for example, about 20 ml to about 30 ml, per 1 g of the compound of Formula 3.

Temperature conditions for the crystallization may be performed at a temperature in a range of about 0 °C to about 50 °C, for example, about 10 °C to about 30 °C, for about 1 hour to about 24 hours, for example, about 8 hours to about 12 hours.

The preparation method according to the present disclosure includes **step 2)** of obtaining a compound of Formula 2 through a reaction between the compound of Formula 3 and acid in an organic solvent.

In one embodiment, the compound of Formula 2 may be a acid salt of 4-(3-aminophenoxy)-N-(4-(4-methylpiperazine-1-yl)phenyl)thieno[3,2-d]pyrimidine-2-amine. In one embodiment, the compound of Formula 2 may be 4-(3-aminophenoxy)-N-(4-(4-methylpiperazine-1-yl)phenyl)thieno[3,2-d]pyrimidine-2-amine trihydrochloride or 4-(3-aminophenoxy)-N-(4-(4-methylpiperazine-1-yl)phenyl)thieno[3,2-d]pyrimidine-2-amine ditrifluoroacetic acid.

In one embodiment regarding step 2), the acid in Formula 2 may be inorganic acid or organic acid. The inorganic acid may be selected from the group consisting of hydrochloric acid, nitric acid, sulfuric acid, and any combination thereof. In addition, the organic acid may be selected from the group consisting of toluenesulfonic acid, benzene sulfonic acid, methanesulfonic acid, acetic acid, trifluoroacetic acid, and any combination thereof. In addition, the acid in Formula 2 may be hydrochloric acid or trifluoroacetic acid.

In addition, an amount of the acid used in the reaction above may be in a range of about 3.0 moles to about 15.0 moles, for example, about 5.0 moles to about 10.0 moles, per equivalent weight of 1 mole of the compound of Formula 3. For example, the acid used in the reaction above may be trimolecular hydrochloric acid or bimolecular trifluoroacetic acid.

In one embodiment regarding step 2), t-butyl(3-((2-((4-(4-methylpiperazine-1-yl)phenyl)amino)thieno[3,2-d]pyrimidine-4-yl)oxy)phenyl)carbamate, which is an example of the compound of Formula 3, is reacted with hydrochloric acid in the presence of an organic solvent, and a cooling process is performed thereon for crystallization. The resulting product is filtered to obtain 4-(3-aminophenoxy)-N-(4-(4-methylpiperazine-1-yl)phenyl)thieno[3,2-d]pyrimidine-2-amine trihydrochloride, which is an example of the compound of Formula 2.

In one or more embodiments regarding step 2), t-butyl(3-((2-((4-(4-methylpiperazine-1-yl)phenyl)amino)thieno[3,2-d]pyrimidine-4-yl)oxy)phenyl)carbamate, which is an example of the compound of Formula 3 is reacted with trifluoroacetic acid in the presence of an organic solvent, and a cooling process is performed thereon for crystallization. The resulting product is filtered to obtain 4-(3-aminophenoxy)-N-(4-(4-methylpiperazine-1-yl)phenyl)thieno[3,2-d]pyrimidine-2-amine ditrifluoroacetic acid, which is an example of the compound of Formula 2.

In one or more embodiments regarding step 2), benzyl(3-((2-((4-(4-methylpiperazine-1-yl)phenyl)amino)thieno[3,2-d]pyrimidine-4-yl)oxy)phenyl)carbamate, which is an example of the compound of Formula 3, is reacted with hydrochloric acid in the presence of an organic solvent, and a cooling process is performed thereon for crystallization. The resulting product is filtered to obtain 4-(3-aminophenoxy)-N-(4-(4-methylpiperazine-1-yl)phenyl)thieno[3,2-d]pyrimidine-2-amine trihydrochloride, which is an example of the compound of Formula 2.

In one or more embodiments regarding step 2), benzyl(3-((2-((4-(4-methylpiperazine-1-yl)phenyl)amino)thieno[3,2-d]pyrimidine-4-yl)oxy)phenyl)carbamate, which is an example of the compound of Formula 3, is reacted with trifluoroacetic acid in the presence of an organic solvent, and a cooling process is performed thereon for crystallization. The resulting product is filtered to obtain 4-(3-aminophenoxy)-N-(4-(4-methylpiperazine-1-yl)phenyl)thieno[3,2-d]pyrimidine-2-amine ditrifluoroacetic acid, which is an example of the compound of Formula 2.

The organic solvent used in the reaction above may be selected from acetone, tetrahydrofuran, dioxane, acetonitrile, methanol, ethanol, butanol, 2-butanol, isopropanol, N'N-dimethylformamide, N'N'-dimethylacetamide, dimethylsulfoxide, and any combination thereof. In one embodiment, the organic solvent may be ethanol.

An amount of the organic solvent may be in a range of about 5 ml to about 30 ml, for example, about 12 ml to about 15 ml, per 1 g of the compound of Formula 3.

The reaction above may be performed at a temperature in a range of about 10 °C to about 100 °C, for example, about 40 °C to about 70 °C, for about 1 hour to about 5 hours, for example, about 2 hours to about 3 hours.

The compound of Formula 2 resulting from step 2) may be obtained in the form of a crystal by adding water or a mixed solvent of an organic solvent and water to a reactant in which the compound of Formula 2 is present and precipitating the resulting product in a solid state.

The organic solvent used for the crystallization may be an organic solvent mixable with water, and for example, may be selected from the group consisting of acetone, methanol, ethanol, isopropanol, acetonitrile, and any combination thereof. In one embodiment, the organic solvent may be acetone or isopropanol.

A mixing ratio of the organic solvent and water may vary depending on the type of the organic solvent. In one embodiment, when the organic solvent is acetone or isopropanol, the mixing ratio of the organic solvent and water may be in a range of about 1:10 to about 10:1, for example, about 3:1 to about 1:3, by volume. In one embodiment, the mixing ratio may be in a range of about 1:1 to about 1:2 by volume.

An amount of water or the mixed solvent of the organic solvent mixable with water used for the crystallization may be in a range of about 10 ml to about 50 ml, for example, about 20 ml to about 30 ml, per 1 g of the compound of Formula 2.

Temperature conditions for the crystallization may be performed at a temperature in a range of about 0 °C to about 50 °C, for example, about 10 °C to about 25 °C, for about 1 hour to about 24 hours, for example, about 8 hours to about 12 hours.

The preparation method according to the present disclosure includes **step 3)** of obtaining N-(3-((2-((4-(4-methylpiperazine-1-yl)phenyl)amino)thieno[3,2-d]pyrimidine-4-yl)oxy)phenyl)acrylamide of Formula 1 through a reaction between the compound of Formula 2 and acryloyl chloride in the presence of a base in an organic solvent.

In one embodiment regarding step 3), 4-(3-aminophenoxy)-N-(4-(4-methylpiperazine-1-yl)phenyl)thieno[3,2-d]pyrimidine-2-amine trihydrochloride, which is an example of the compound of Formula 2, is reacted with acryloyl chloride in the presence of a base in an organic solvent. Then, water or a mixed solvent of an organic solvent and water is added thereto for crystallization, and the resulting product is filtered to obtain N-(3-((2-((4-(4-methylpiperazine-1-yl)phenyl)amino)thieno[2,3-d]pyrimidine-4-yl)oxy)phenyl)acrylamide, which is an example of the compound of Formula 1.

In one or more embodiments regarding step 3), 4-(3-aminophenoxy)-N-(4-(4-methylpiperazine-1-yl)phenyl)thieno[3,2-d]pyrimidine-2-amine ditrifluoroacetic acid, which is an example of the compound of Formula 2, is reacted with acryloyl chloride in the presence of a base in an organic solvent. Then, water or a mixed solvent of an organic solvent and water is added thereto for crystallization, and the resulting product is filtered to obtain N-(3-((2-((4-(4-methylpiperazine-1-yl)phenyl)amino)thieno[2,3-d]pyrimidine-4-yl)oxy)phenyl)acrylamide, which is an example of the compound of Formula 1.

The organic solvent used in the reaction with the compound of Formula 2 may be selected from the group consisting of acetone, acetonitrile, methyl ethyl ketone, methyl isobutyl ketone, methyl acetate, ethyl acetate, isopropyl acetate, N'N'-dimethylformamide, N'N-dimethylacetamide, dimethylsulfoxide, and any mixed solvent thereof. In one embodiment, the organic solvent may be acetone.

An amount of the organic solvent may be in a range of about 5 ml to about 30 ml, for example, about 15 ml to about 20ml, per 1 g of the compound of Formula 2.

The organic solvent may be mixed with water. Here, a mixing ratio of the organic solvent and water may vary depending on the type of the organic solvent. In one embodiment, when the organic solvent is acetone, the mixing ratio of the organic solvent and water may be in a range of about 1:10 to about 10:1, for example, about 5:1 to about 1:5, by volume. In one embodiment, the mixing ratio may be in a range of about 4:1 by volume.

In addition, the base used in the reaction above may be selected from the group consisting of potassium carbonate, calcium carbonate, sodium carbonate, potassium hydroxide, sodium hydroxide, potassium bicarbonate, sodium bicarbonate, and any mixture thereof. In one embodiment, the base may be potassium bicarbonate or sodium bicarbonate.

An amount of the base may be in a range of about 3.0 moles to about 10.0 moles, for example, about 5.0 moles to about 7.moles, per equivalent weight of 1 mole of the compound of Formula 2.

The reaction above may be performed at a temperature in a range of about -5 °C to about 10 °C, for example, about 0 °C to about 10°C, for about 0.1 hour to about 3 hours, for example, about 0.5 hour to about 1 hour.

The compound of Formula 1 resulting from step 3) may be obtained in the form of a crystal by adding water or a mixed solvent of an organic solvent and water to a reactant in which the compound of Formula 1 is present and precipitating the resulting product in a solid state.

The organic solvent used for the crystallization may be an organic solvent mixable with water, and for example, may be selected from the group consisting of acetone, methanol, ethanol, isopropanol, acetonitrile, and any combination thereof. In one embodiment, the organic solvent may be acetone or isopropanol.

A mixing ratio of the organic solvent and water may vary depending on the type of the organic solvent. In one embodiment, when the organic solvent is acetone or isopropanol, the mixing ratio of the organic solvent and water may be in a range of about 1:10 to about 10:1, for example, about 3:1 to about 1:3, by volume. In one embodiment, the mixing ratio may be in a range of about 1:1 to about 1:2 by volume.

An amount of the mixed solvent of the organic solvent and water used for the crystallization may be in a range of about 10 ml to about 50 ml, for example, about 15 ml to about 30 ml, per 1 g of the compound of Formula 1.

Temperature conditions for the crystallization may be performed at a temperature in a range of about 0 °C to about 35 °C, for example, about 10 °C to about 25 °C, for about 1 hour to about 24 hours, for example, about 8 hours to about 12 hours.

The preparation method according to the present disclosure may further include a process of separating an organic solvent layer in which the compound of Formula 1 is present, after the reaction above.

The organic solvent which can be used for the separation of the organic solvent layer may be selected from chloroform, dichloromethane, and a mixture thereof. The organic solvent may be used alone or in combination with water. Here, a mixing ratio of the organic solvent and water may vary depending on the type of the organic solvent. In one embodiment, when the organic solvent is chloroform or dichloromethane, the mixing ratio of water and the organic solvent may be in a range of about 1:1 to about 1:5, for example, about 1:1 to about 1:3, by volume. The separating of the organic solvent layer may be repeated one or more times, specifically, one to three times.

The compound of Formula 1 resulting from the reaction above may be obtained in the form of a crystal by adding water or the organic solvent to a reactant in which the compound of Formula 1 is present and precipitating the resulting product in a solid state.

The organic solvent may be mixed with water. Here, a mixing ratio of the organic solvent and water may vary depending on the type of the organic solvent. In one embodiment, when the organic solvent is acetone or isopropanol, the mixing ratio of the organic solvent and water may be in a range of about 1:3 to about 1:9, for example, about 1:3 to about 1:6, by volume. In one embodiment, the mixing ratio may be in a range of about 1:3 to about 1:6 by volume.

In addition, the preparation method according to the present disclosure may further include a process of increasing purity of the compound of Formula 1 obtained by the method above. To increase the purity, either or both of the following two methods may be used.

As a first method for increasing the purity, a mixed solvent of an organic solvent and water is added to the compound of Formula 1, followed by being heated up to a reflux temperature of the mixed solvent and stirred for dissolution. Then, the resulting product is cooled to room temperature, and a re-precipitated solid product is filtered and dried, thereby obtaining the compound of Formula 1 that is further purified.

As a second method for increasing the purity, before a mixed solvent of an organic solvent and water is added to precipitate the resulting mixed solvent in a solid state, an organic solvent is added to a reaction solution which is reacted with the compound of Formula 2, an insoluble product generated by heating the reaction solution is removed by filtration, an organic solvent is added thereto, and then, the resulting mixture is cooled, thereby obtaining the compound of Formula 1 which is further purified by precipitation as described above.

The organic solvent which can be used in the process of increasing the purity may be the same as the organic solvent which can be used for the precipitation of the compound. The organic solvent may be used alone or in combination with water. Examples of the organic solvent include acetone, methanol, ethanol, isopropanol, acetonitrile, and the like. In one embodiment, the organic solvent may be acetone or isopropanol. When the organic solvent is used in combination with water, a mixing ratio of the organic solvent and water may vary depending on the type of the organic solvent. In one or more embodiments, when the organic solvent is acetone or isopropanol, a mixing ratio of the organic solvent and water may be in a range of about 8:1 to about 2:1, for example, about 5:1 to about 3:1, by volume. In one embodiment, the mixing ratio may be about 4:1 by volume.

Reaction conditions for the dissolution in the reaction above may be performed at a temperature of about 50 °C or higher, for example, at a reflux temperature of the solvent within 50 °C, for about 0.5 hour to about 3 hours, for example, about 1 hour to about 2 hours. In addition, the cooling in the re-precipitation step may be performed at room temperature or lower, for example, at room temperature within 5 °C, for about 2 hours to about 24 hours, for example, about 8 hours to about 12 hours. Here, the re-precipitation step may be repeated one or more times, specifically, two or more times.

An apparatus and a specific measuring method for analyzing the compounds of the present disclosure are as follows.

### (1) (1) High-Performance Liquid Chromatography (HPLC)

For the purpose of analyzing the purity and amount through the stability test or the like, an Agilent 1100/1200 series HPLC Systems (Agilent, USA) analyzing device is used to perform HPLC analysis, and conditions for HPLC analysis are as follows.

### Conditions for analyzing purity and amount: Thienopyrimidine compound of Formula 1

Column: Hydrosphere C18 (YMC), 5 *µ*m (150 mm × 4.6 mm)
Column temperature: 30 °C
Detector: Ultraviolet spectrophotometer
Detection wavelength: 254 nm
Velocity: 1.0 ml/min
Time of analysis: 35 minutes
Solution A: pH 3.0 phosphate buffer solution [NaClO₄7g + NaH₂PO₄-1.7g/L]
Solution B: 100% acetonitrile

**[Table 1]**

| Time (minutes) | Solution A (%) | Solution B (%) |
|---|---|---|
| 0 → 5 | 65 | 35 |
| 5 → 25 | 65 → 30 | 35 → 70 |
| 25 → 27 | 30 → 65 | 70 → 35 |
| 27 → 35 | 65 | 35 |

### (2) Ion Chromatography (IC)

For the purpose of analyzing amount of hydrochloric acid of hydrochloride, a Thermo Fisher Scientific ICS-2500 series IC Systems (Thermo Fisher Scientific, USA) analyzing device is used to perform IC, and conditions for IC are as follows.

### Conditions for analyzing amount: Thienopyrimidine compound of Formula 1

Column: IonPac AS19 (Dionex), (250 mm × 4 mm), guard (50 mm × 4 mm)
Column temperature: 30 °C
Detector: Conductivity detector (CD)
Suppressor: ASRS 4mm, current 40mA
Injection volume: 25 *µ*ℓ
Velocity: 1.0 ml/min
Time of analysis: 30 minutes
Eluant: 10 mM potassium hydroxide solution
Sample concentration: 0.5 mg/ml 50 % acetonitrile (0.1 N sodium hydroxide 8 ml)

### (3) High-Resolution Mass Spectrometry (LC/MS)

A Waters Acquity UPLC/Synapt G2 QTOF MS (USA) analyzing device is used for MS, and conditions for MS are as follows.

### Conditions for analyzing High-Resolution MS: Thienopyrimidine compound of Formula 1

Column: Acquity CSH C18, 1.7 *µ*m (150 mm × 2.1 mm ID)
Column temperature: 30 °C
Velocity: 0.3 ml/min
Injection volume: 1 *µ*ℓ (0.5 mg/ml)
Detection method and MS parameters:
ESI positive mode, MSE 20^{∼}40V, Scan time: 0.5s
Capillary (kV): 2.5
Sampling Cone (V): 25
Extraction Cone (V): 4
Source temp (°C): 100
Desolvation temp (°C): 500
Cone gas (L/h): 0
Desolvation gas (L/h): 700
Time of analysis: 40 minutes
Solution A: 10 mM ammonium formate (pH 3.5)
Solution B: 100% acetonitrile

**[Table 2]**

| Time (minutes) | Solution A (%) | Solution B (%) |
|---|---|---|
| 0 → 5 | 90 | 10 |
| 5 → 15 | 90 → 50 | 10 → 50 |
| 15 → 30 | 50 → 30 | 50 → 70 |
| 30 → 40 | 30 | 70 |

### (4) Measurement of melting point

An IA9200 (Electrothermal, UK) melting point meter is used to measure a melting point.

### Conditions for measurement of melting point: Thienopyrimidine compound of Formula 1

Initiation temperature: 150 °C
Heating rate: 5 °C/min

The compounds of Formulae 4 and 5 which are used as starting materials in the methods described above can be commercially obtained, manufactured according to conventional methods, or prepared according to the following preparation examples. However, the following preparation examples are for illustrating the preparation method of the starting materials used in the present disclosure, and the present disclosure is not limited thereto.

### Preparation Example 1. Preparation of t-butyl (3-hydroxyphenyl)carbamate

10 g (0.0916 mol) of 3-aminopenol was dissolved in 500 ml of tetrahydrofuran, and 27 ml (0.119 mol) of di-t-butyl dicarbonate was added thereto. The mixed solution was then stirred at a temperature of 25 °C (room temperature) for 1 hour. After completion of the reaction, a mixed solvent of 25 ml of isopropyl alcohol and 50 ml of hexane was added to the residue resulting from removal of the solvent by distillation under reduced pressure, and the resulting solution was stirred for 2 hours. The resulting solid was filtered, washed with 50 ml of hexane, and dried at a temperature of 40 °C, thereby obtaining 17 g (yield: 90 %) of t-butyl (3-hydroxyphenyl)carbamate which is the title compound as a white solid.

¹H-NMR(300 MHz, DMSO-d₆) δ 9.25(s, 1 H), 9.18(s, 1 H), 7.01(m, 1 H), 6.97(d, 1 H), 6.83(dd, 1 H), 6.34(m, 1 H), 1.45(s, 9 H)

### Preparation Example 2. Preparation of 4-chloro-N-(4-(4-methylpiperazine-1-yl)phenyl)thieno[3,2-d]pyrimidine-2-amine

150 g (0.44 mol) of 2-((4-(4-methylpiperazine-1-yl)phenyl)amino)thieno[3,2-d]pyrimidine-4(3 H)-on was dissolved in 600 ml of acetonitrile while a reaction temperature was raised up to 75 °C, and a solution in which 124 ml of phosphorus oxychloride (POCl₃) was diluted in 150 ml of acetonitrile was added thereto. The mixed solution was then stirred at a temperature of 75 °C for 1 hour. After completion of the reaction, the reaction temperature was cooled down to a temperature of 25 °C, 1.5 ℓ of cooling water at a temperature of 0 °C to 4 °C was slowly added to the reaction solution. Afterwards, an aqueous solution prepared by dissolving 263 g of sodium hydroxide in 750 ml of water was added to the reaction solution while the reaction temperature was maintained at a temperature of 20 °C or less, and the resulting reaction solution was stirred at a temperature of 25 °C for 1 hour. The resulting solid was filtered, washed with 1.5 ℓ, of water, and dried at a temperature of 50 °C, thereby obtaining 180 g (yield: 114 %) of a primary crude product of the title compound.

180 g of the primary crude product was dissolved in 3.0 ℓ of a mixed solvent in which dichloromethane and methanol were mixed at a ratio of 3:2, and the resulting solution was stirred at a temperature of 25 °C for 1 hour. Afterwards, 1.5 ℓ of a mixed solvent of acetone (80 %) and water (20 %) was added to the residue in which the stirring product was subjected to diatomaceous earth filtration to remove impurities and to distillation under reduced pressure. The resulting solution was stirred at a temperature of 25 °C for 2 hours. The resulting solid was filtered and dried at a temperature of 50 °C, thereby obtaining 125 g (yield: 79 %) of 4-chloro-N-(4-(4-methylpiperazine-1-yl)phenyl)thieno[3,2-d]pyrimidine-2-amine which is the title compound as a yellow solid.

Melting point: 179 °C to 181°C
MS spectrum: m/z=360.11(M+1)

Hereinafter, the present disclosure will be described in detail with reference to Examples. However, the following Examples are intended to be illustrative of the present disclosure only, and the present disclosure is not limited thereto.

### Example 1. Preparation of N-(3-((2-((4-(4-methylpiperazine-1-yl)phenyl)amino)thieno[3,2-d]pyrimidine-4-yl)oxy)phenyl)acrylamide (1)

### 1.1. Preparation of t-butyl(3-((2-((4-(4-methylpiperazine-1-yl)phenyl)amino)thieno[3,2-d]pyrimidine-4-yl)oxy)phenyl)carbamate

7.0 g (0.0278 mol) of t-butyl (3-hydroxyphenyl)carbamate obtained in Preparation Example 1, 10.0 g (0.0278 mol) of 4-chloro-N-(4-(4-methylpiperazine-1-yl)phenyl)thieno[3,2-d]pyrimidine-2-amine, and 11.5 g (0.0333 mol) of potassium carbonate were dissolved in 100 ml of dimethylsulfoxide, and the mixed solution was stirred for 6 hours while being heated at a temperature of 60 °C.

After completion of the reaction, the reaction temperature was cooled down to a temperature of 25 °C, and 200 ml of water at a temperature of 25 °C was slowly added to the reaction solution. Afterwards, the mixed solution was stirred for 12 hours at a temperature of 25 °C. The resulting solid was filtered, washed with 200 Im of water, and dried at a temperature of 50 °C, thereby obtaining 14.0 g (yield: 95%) of t-butyl(3-((2-((4-(4-methylpiperazine-1-yl)phenyl)amino)thieno[3,2-d]pyrimidine-4-yl)oxy)phenyl)carbamate which is the title compound as a yellow solid.

Melting point: 180 °C to 183 °C
MS spectrum: m/z=533.23(M+1)
^{1H}-NMR(300 MHz, DMSO-d₆) δ 9.58(s, 1 H), 9.22(s, 1 H), 8.25(d, 1 H), 7.46-7.36(m, 5 H), 7.30(d, 1 H), 6.94^{∼}6.91(m, 1 H), 6.72(d, 2 H), 3.00(m, 4 H), 2.43(m, 4 H), 2.20(s, 3 H), 1.45(s, 9 H)

### 1.2. Preparation of 4-(3-aminophenoxy)-N-(4-(4-methylpiperazine-1-yl)phenyl)thieno[3,2-d]pyrimidine-2-amine trihydrochloride

14.0 g (0.0263 mol) of t-butyl(3-((2-((4-(4-methylpiperazine-1-yl)phenyl)amino)thieno[3,2-d]pyrimidine-4-yl)oxy)phenyl)carbamate obtained in Example 1.1 was added to 280 ml of ethanol, and 22 ml (0.263 mol) of concentrated hydrochloric acid was added thereto. The mixed solution was stirred for 2 hours while being heated at a temperature of 60 °C. After completion of the reaction, the reaction temperature was cooled down to a temperature of 25 °C, and the resulting solution was stirred for 12 hours. The resulting solid was filtered, washed with 70 ml of ethanol, and dried at a temperature of 50 °C, thereby obtaining 11.5 g (yield: 81 %) of 4-(3-aminophenoxy)-N-(4-(4-methylpiperazine-1-yl)phenyl)thieno[3,2-d]pyrimidine-2-amine trihydrochloride which is the title compound as a yellow solid.

Melting point: 255 °C to 260 °C
MS spectrum: m/z=433.18(M+1)
Measured value by IC: 20.3 % (theoretical value: 20.2 %)
^{1H}-NMR(300 MHz, DMSO-d₆) δ 11.05(br, 1 H), 9.69(s, 1 H), 8.35(d, 1 H), 7.60(t, 1 H), 7.45-7.35(m, 6H), 6.89-6.86(d, 2 H), 3.72(m, 2 H), 3.48-3.39(m, 2 H), 3.18-3.01(m, 4 H), 2.79(s, 3 H)

### 1.3. Preparation of N-(3-((2-((4-(4-methylpiperazine-1-yl)phenyl)amino)thieno[3,2-d]pyrimidine-4-yl)oxy)phenyl)acrylammide by extraction

1.0 g (1.84 mmol) of 4-(3-aminophenoxy)-N-(4-(4-methylpiperazine-1-yl)phenyl)thieno[3,2-d]pyrimidine-2-amine trihydrochloride obtained in Example 1.2 and 0.93 g (11.07 mmol) of sodium bicarbonate were dissolved in 16.7 ml of tetrahydrofuran and 3.3 ml of water, and 0.164 ml (2.03 mmol) of acryloyl chloride was slowly added thereto at a temperature in a range of about 0 °C to about 5 °C. The mixed solution was then stirred for 30 minutes. After completion of the reaction, 30 ml of water was added thereto, and an extraction process was performed thereon using 40 ml of ethylacetate. An organic layer extracted therefrom was washed with a saturated aqueous solution of sodium chloride, and then, subjected to distillation under reduced pressure, thereby obtaining 0.63 g (yield: 70 %) of N-(3-(2-(4-(4-methylpiperazine-1-yl)phenylamino)thieno[2,3-d]pyrimidine-4-yl)oxy)phenyl)acrylamide which is the title compound.

Melting point: 193 °C to 195 °C

### Example 2. Preparation of N-(3-((2-((4-(4-methylpiperazine-1-yl)phenyl)amino)thieno[3,2-d]pyrimidine-4-yl)oxy)phenyl)acrylamide (2)

### 2.1. Preparation of t-butyl(3-((2-((4-(4-methylpiperazine-1-yl)phenyl)amino)thieno[3,2-d]pyrimidine-4-yl)oxy)phenyl)carbamate

7.0 g (0.0278 mol) of t-butyl (3-hydroxyphenyl)carbamate obtained in Preparation Example 1, 10.0 g (0.0278 mol) of 4-chloro-N-(4-(4-methylpiperazine-1-yl)phenyl)thieno[3,2-d]pyrimidine-2-amine, and 11.5 g (0.0333 mol) of potassium carbonate were dissolved in 100 ml of dimethylsulfoxide. The mixed solution stirred for 6 hours while being heated at a temperature of 60 °C.

After completion of the reaction, the reaction temperature was cooled down to a temperature of 25 °C, and 200 ml of water at a temperature of 25 °C was slowly added to the reaction solution. Afterwards, the resulting reaction solution was stirred at a temperature of 25 °C for 12 hours. The resulting solid was filtered, washed with 200 ml of water, and dried at a temperature of 50 °C, thereby obtaining 14.0 g (yield: 95 %) of t-butyl(3-((2-((4-(4-methylpiperazine-1-yl)phenyl)amino)thieno[3,2-d]pyrimidine-4-yl)oxy)phenyl)carbamate which is the title compound as a yellow solid.
Melting point: 180 °C to 183 °C
MS spectrum: m/z=533.23(M+1)
^{1H}-NMR(300 MHz, DMSO-d₆) δ 9.58(s, 1 H), 9.22(s, 1 H), 8.25(d, 1 H), 7.46-7.36(m, 5 H), 7.30(d, 1 H), 6.94^{∼}6.91(m, 1 H), 6.72(d, 2 H), 3.00(m, 4 H), 2.43(m, 4 H), 2.20(s, 3 H), 1.45(s, 9 H)

### 2.2. Preparation of 4-(3-aminophenoxy)-N-(4-(4-methylpiperazine-1-yl)phenyl)thieno[3,2-d]pyrimidine-2-amine ditrifluoroacetic acid

10.0 g (0.0188 mol) of tert-butyl(3-((2-((4-(4-methylpiperazine-1-yl)phenyl)amino)thieno[3,2-d]pyrimidine-4-yl)oxy)phenyl)carbamate obtained in Example 2.1 was added to 150 ml of ethanol, and then, 7.2 ml (0.0939 mol) of trifluoroacetic acid was added thereto at room temperature. The mixed solution was stirred for 2 hours while being heated at a temperature of 60 °C. After completion of the reaction, the reaction temperature was cooled down to a temperature of 25 °C, and the resulting reaction solution was stirred for 12 hours. The resulting solid was filtered, washed with 50 ml of ethanol, and dried at a temperature of 50 °C, thereby obtaining 8.0 g (yield: 78 %) of 4-(3-aminophenoxy)-N-(4-(4-methylpiperazine-1-yl)phenyl)thieno[3,2-d]pyrimidine-2-amine ditrifluoroacetic acid which is the title compound as a yellow solid.

Melting point: 206 °C to 210 °C
MS spectrum: m/z=433.18(M+1)
Measured value by IC: 35.9 % (theoretical value: 34.5 %)
^{1H}-NMR(300 MHz, DMSO-d6) δ 9.91(br, 1 H), 9.33(s, 1 H), 8.25(d, 1 H), 7.57-7.54(d, 2 H), 7.30(d, 1 H), 7.22-7.17(m, 1 H), 6.86-6.83(d, 2 H), 6.67-6.64(d, 1 H), 6.61-6.59(m, 2 H), 3.70-3.66(m, 2 H), 3.53-3.49(m, 2 H), 3.16(m, 2 H), 2.91(m, 2 H), 2.86(s, 3 H)

### 2.3. Preparation of N-(3-((2-((4-(4-methylpiperazine-1-yl)phenyl)amino)thieno[3,2-d]pyrimidine-4-yl)oxy)phenyl)acrylamide by crystallization

11.5 g (0.0212 mol) of 4-(3-aminophenoxy)-N-(4-(4-methylpiperazine-1-yl)phenyl)thieno[3,2-d]pyrimidine-2-amine ditrifluoroacetic acid obtained in Example 2.2 and 10.7 g (0.127 mol) of sodium bicarbonate were dissolved in 184 ml of acetone and 46 ml of water, and 1.9 ml (0.0233 mol) of acryloyl chloride was slowly added thereto at a temperature in a range of about 0 °C to about 5 °C. The mixed solution was then stirred for 30 minutes. After completion of the reaction, 172.5 ml of water was added thereto at a temperature in a range of about 0 °C to about 5 °C, and the resulting reaction solution was stirred for about 1 hour until a solid was precipitated. The resulting reaction solution was stirred again for 12 hours at a temperature of 25 °C. The resulting solid was filtered, washed with 115 ml of water, and dried at a temperature of 50 °C, thereby obtaining 7.7 g (yield: 75 %) of N-(3-((2-((4-(4-methylpiperazine-1-yl)phenyl)amino)thieno[3,2-d]pyrimidine-4-yl)oxy)phenyl)acrylamide which is the title compound as a yellow solid.

Melting point: 198 °C to 201 °C
MS spectrum: m/z=487.19(M+1)
^{1H}-NMR(300 MHz, DMSO-d₆) δ 10.37(s, 1 H), 9.24(s, 1 H), 8.27(d, 1 H), 7.71(d, 1 H), 7.64(d, 1 H), 7.49-7.41(m, 3 H), 7.32(d, 1 H), 7.07(dd, 1 H), 6.71(d, 2 H), 6.42(dd, 1 H), 6.28(dd, 1 H), 5.78(dd, 1 H), 2.99(t, 4 H), 2.43(t, 4 H), 2.21(s, 3 H)

## Claims

1. A method of preparing a compound of Formula 1, the method comprising:
1) obtaining a compound of Formula 3 through a reaction between a compound of Formula 4 and a compound of Formula 5 in the presence of a base in an organic solvent;
2) obtaining a compound of Formula 2 through a reaction between the compound of Formula 3 and acid in an organic solvent; and
3) performing a reaction between the compound of Formula 2 and acryloyl chloride in the presence of a base in an organic solvent: 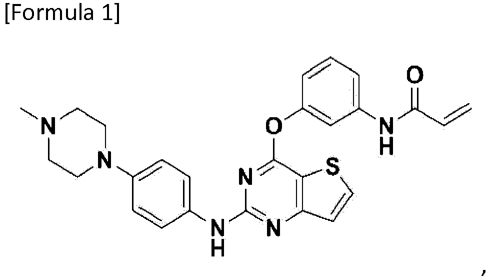 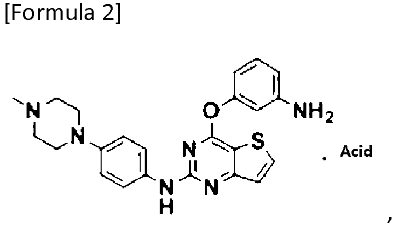 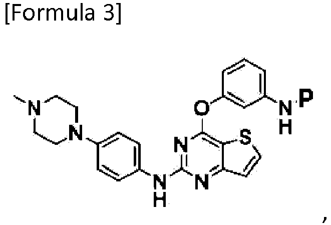 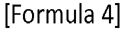 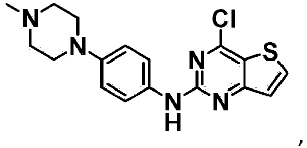 and 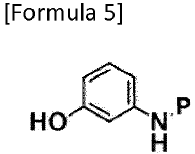 wherein P in Formulae 3 and 5 represents an amino group-protecting group.

2. The method of claim 1, wherein the organic solvent used in step 1) is one selected from the group consisting of acetonitrile, acetone, methyl ethyl ketone, methyl isobutyl ketone, methyl acetate, ethyl acetate, isopropyl acetate, N'N'-dimethylformamide, N'N-dimethylacetamide, dimethylsulfoxide, and any combination thereof.

3. The method of claim 1, wherein the base used in step 1) is one selected from the group consisting of potassium carbonate, calcium carbonate, sodium carbonate, potassium hydroxide, sodium hydroxide, sodium bicarbonate, and any mixture thereof.

4. The method of claim 1, wherein the method further comprises, after the reaction of step 1), a process of precipitating the compound of Formula 3 by adding water or a mixed solvent of an organic solvent and water to a solution in which the compound of Formula 3 is present.

5. The method of claim 4, wherein the organic solvent is selected from the group consisting of acetone, methanol, ethanol, isopropanol, acetonitrile, and any combination thereof.

6. The method of claim 5, wherein the organic solvent is acetone or isopropanol.

7. The method of claim 4, wherein the mixed solvent comprises the organic solvent and water at a ratio of about 3:1 to about 1:3 by volume.

8. The method of claim 1, wherein the organic solvent used in step 2) is selected from the group consisting of tetrahydrofuran, dioxane, acetonitrile, methanol, ethanol, butanol, 2-butanol, isopropanol, N'N-dimethylformamide, N'N'-dimethylacetamide, dimethylsulfoxide, and any combination thereof.

9. The method of claim 1, wherein the acid used in step 2) is inorganic acid or organic acid.

10. The method of claim 9, wherein the inorganic acid is one selected from group consisting of hydrochloric acid, nitric acid, sulfuric acid, and any combination thereof.

11. The method of claim 9, wherein the organic acid is one selected from group consisting of toluenesulfonic acid, benzene sulfonic acid, methanesulfonic acid, acetic acid, trifluoroacetic acid, and any combination thereof.

12. The method of claim 10, wherein the inorganic acid is hydrochloric acid.

13. The method of claim 11, wherein the organic acid is trifluoroacetic acid.

14. The method of claim 1, wherein the organic solvent used in step 3) is one selected from the group consisting of acetonitrile, acetone, methyl ethyl ketone, methyl isobutyl ketone, methyl acetate, ethyl acetate, isopropyl acetate, N'N'-dimethylformamide, N'N-dimethylacetamide, dimethylsulfoxide, and any mixture thereof.

15. The method of claim 1, wherein the base used in step 3) is one selected from the group consisting of potassium carbonate, calcium carbonate, sodium carbonate, potassium hydroxide, sodium hydroxide, sodium bicarbonate, and any mixture thereof.

16. The method of claim 1, wherein the method further comprises, after the reaction of step 3), a process of precipitating the compound of Formula 1 by adding water or a mixed solvent of an organic solvent and water to a solution in which the compound of Formula 1 is present.

17. The method of claim 16, wherein the organic solvent is one selected from the group consisting of acetone, methanol, ethanol, isopropanol, acetonitrile, and any combination thereof.

18. The method of claim 16, wherein the organic solvent is acetone or isopropanol.

19. The method of claim 16, wherein the mixed solvent comprises the organic solvent and water at a ratio of about 3:1 to about 1: 3 by volume.

20. A compound of Formula 2:

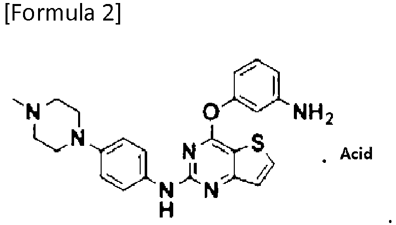

21. The compound of claim 20, wherein the acid is hydrochloric acid or trifluoroacetic acid.

22. A compound of Formula 3: 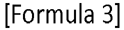 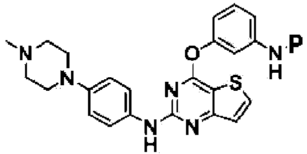 wherein P in Formula 3 represents an amino group-protecting group.

23. The compound of claim 22, wherein P is a tert-butyloxycarbonyl group (Boc) or a benzyloxycarbonyl group (Cbz).
